# EUROPEAN PATENT APPLICATION

(11) **EP 4 098 244 A1**
(43) Date of publication of application: **07.12.2022**
(21) Application number: 21176938.5
(22) Date of filing: 31.05.2021
(51) Int. Cl.: A61K 8/31, A61K 8/34, A61K 8/36, A61K 8/37, A61K 8/39, A61K 8/44, A61K 8/92, A61Q 9/02, B26B 21/44

(54) **SOLID SHAVING AID COMPOSITION**

(71) Applicant: BIC Violex Single Member S.A., 14569 Anoixi (GR)
(72) Inventor: Zanidaki, Maria-Thiresia, 145 69 Anoixi (GR); Vlachos, Georgios, 145 69 Anoixi (GR)
(74) Representative: Peterreins Schley

(57) **Abstract**

The present disclosure relates to a razor cartridge comprising a housing having a front edge and a rear edge. The housing comprises one or more shaving blades disposed between the front edge and the rear edge. At least one shaving bar is disposed in front of the blade(s) and/or rear of the blade(s). The shaving bar comprises a solid shaving aid composition. The solid shaving aid composition may comprise, relative to the weight of the solid shaving aid composition: at about 25 wt.-% to about 75 wt.-% of a soap base consisting of one or more fatty acid salts having from about 8 to about 24 carbon atoms and optionally one or more fatty acids having from about 8 to about 24 carbon atoms, about 15 wt.-% to about 60 wt.-% of one or more polyols comprising from about 2 to about 8 carbon atoms, about 0.1 wt.-% to about 3.0 wt.-% of one or more non-ionic surfactants, about 0.1 wt.-% to about 3.0 wt.-% of one or more zwitterionic surfactant, one or more butters, and one or more oils.

## Description

### Technical Field

The present invention relates to the field of razor cartridges. More specifically, the present invention relates to razor cartridges comprising solid shaving aid compositions.

### Background

Shavers generally include a handle and a razor cartridge fixedly or releasably attached to the handle. The razor cartridge includes at least one blade for shaving hair. The razor cartridge may also include a solid shaving composition, often in the shape of a shaving aid bar extending parallel to at least one blade. The user holds the handle and repeatedly moves the razor across an area of the body to be shaved, e.g., the face, until hair is removed from the surface of the body. The shaving aid bar may function to aid in shaving and to decrease or prevent skin irritation due to the repeated exposure of the skin to sharp razor blades, in particular by providing a glide film between the razor cartridge and the user's skin.

Razor cartridges comprising such solid shaving compositions are known in the art, for instance from WO 2016/162080 A1. However, while successful in aiding with comfortable and simplified shaving, there is still room for improvement. For instance, current solid shaving compositions, such as those disclosed within WO 2016/162080, may comprise sulfur-containing anionic surfactants as cleansing agents. However, sulfur-containing anionic surfactants may lead to skin irritation. In particular, after multiple uses, the one or more blades of a razor cartridge may have dulled, which may increase the skin irritation during the shaving process itself, and the skin irritation may be amplified by the presence of sulfur-containing anionic surfactants. Therefore, it may be beneficial to provide users with solid shaving compositions comprising limited amounts of sulfur-containing anionic surfactants, while maintaining good cleansing and glide properties.

### Summary

Following diligent research, it was surprisingly found that a new soap-based formulation based on polyols, non-ionic surfactants, zwitterionic surfactants, butter and/or oils is generally advantageous in providing razor cartridges providing sufficient cleansing performance and good glide properties, while reducing skin irritation.

According to a first aspect, the present disclosure relates to a razor cartridge comprising a housing having a front edge and a rear edge; one or more shaving blades disposed between the front edge and the rear edge; and at least one shaving bar disposed in front of the blade(s) and/or rear of the blade(s) which comprises a solid shaving aid composition. The solid shaving aid composition may comprise, relative to the total weight of the solid shaving aid composition: about 25 wt.-% to about 75 wt.-% of a soap base consisting of one or more fatty acid salts having from about 8 to about 24 carbon atoms and optionally one or more fatty acids having from about 8 to about 24 carbon atoms, about 15 wt.-% to about 60 wt.-% of one or more polyols comprising from about 2 to about 8 carbon atoms, about 0.1 wt.-% to about 3.0 wt.-% of one or more non-ionic surfactants, about 0.1 wt.-% to about 3.0 wt.-% of one or more zwitterionic surfactant, one or more butters, and one or more oils.

In some embodiments, the non-ionic surfactant may be selected from polyethoxylated, polypropoxylated and polyglycerolated fatty acids; alkylphenols; copolymers of ethylene oxide and of propylene oxide; condensates of ethylene oxide and of propylene oxide with fatty alcohols; polyethoxylated fatty amides; polyglycerolated fatty amides; polyethoxylated fatty amines; oxyethylenated fatty acid esters of sorbitan; fatty acid esters of sucrose; fatty acid esters of polyethylene glycol; and alkylpolyglycosides.

In some embodiments, the solid shaving aid composition may comprise, relative to the total weight of the solid shaving aid composition, between about 0.2 wt.-% to about 2.5 wt.-% and in particular between about 0.3 wt.-% to about 2.0 wt.-%, of the non-ionic surfactant.

In some embodiments, the at least one zwitterionic surfactant may comprise a positively charged nitrogen-based group, in particular a quaternary ammonium group, and a negatively charged group, in particular a sulfonate or carboxylate group.

In some embodiments, the at least one zwitterionic surfactant may comprise a betaine and/or a sulfobetaine.

In some embodiments, the at least one zwitterionic surfactant may comprise a betaine selected from (C₈-C₂₀)alkylbetaines, sulfobetaines, (C₈-C₂₀)alkylamido(C₁-C₆)alkylbetaines; and (C₈-C₁₀)alkylamido(C₁-C₆)alkylsulfobetaines which may optionally be oxyethylenated.

In some embodiments, the at least one zwitterionic surfactant may be pH-insensitive.

In some embodiments, the at least one zwitterionic surfactant may be present in amount of about 0.2 wt.-% to about 2.5 wt.-%, and in particular in amounts of about 0.3 wt.-% to about 2.0 wt.-%, relative to the total weight of the solid shaving composition.

In some embodiments, the at least one butter may be selected from the group consisting of cocoa butter, mango butter, shea butter and mixtures thereof.

In some embodiments, the total content of butter(s) may be between 0.5 wt.-% to 5.0 wt.-%, in particular about 0.5 % to about 4.0 wt.-%, relative to the total weight of the solid shaving aid composition.

In some embodiments, the one or more oils may comprise a humectant oil having a molecular weight of greater than about 500 g/mol; in particular a castor oil derivative.

In some embodiments, the castor oil derivative may comprise a castor oil esterified with 1, 2 or 3 dicarboxylic acid(s) having between about 3 and about 8 carbon atoms, and in particular comprises castoryl maleate.

In some embodiments, the solid shaving aid composition may comprise, relative to the total weight of the solid shaving aid composition, between about 0.1 wt.-% and about 4.0 wt.-%, more specifically about 0.4 wt.-% to about 3.0 wt.-%, in particular about 0.6 wt.-% to about 2.0 wt.-%, of the one or more oils.

In a further aspect, the present disclosure relates to a solid shaving aid composition having a composition as defined for the first aspect of the present disclosure.

In some embodiments, the solid shaving composition may shaped into the form of a bar, more specifically a bar configured to be placed onto a razor cartridge, and in particular a bar having a length of between about 30 mm to about 80 mm, a width between about 2 mm and about 10 mm, and a depth of about 0.2 mm to about 4 mm.

### Detailed Description

Hereinafter, a detailed description will be given of the present disclosure. The terms or words used in the description and the claims of the present disclosure are not to be construed limitedly as only having common-language or dictionary meanings and should, unless specifically defined otherwise in the following description, be interpreted as having their ordinary technical meaning as established in the relevant technical field. The detailed description will refer to specific embodiments to better illustrate the present disclosure, however, it should be understood that the presented disclosure is not limited to these specific embodiments.

As indicated above, it was surprisingly found that the new soap-based formulation of the present disclosure are generally advantageous in providing razor cartridges providing sufficient cleansing performance and gliding properties, while reducing the skin irritation potential.

Without wishing to be bound by theory, it is believed that the combination of non-ionic and zwitterionic surfactant, embedded in a matrix of fatty acids and fatty acid salts, is providing good cleansing properties, despite the substantial reduction or even exclusion of sulfur-containing anionic surfactants. The non-ionic and zwitterionic surfactants may be mild surfactants and thus, may lead to less skin irritation, compared to sulfur-containing anionic surfactants. At the same time, the non-ionic and zwitterionic surfactants may enable efficient cleansing performance while avoiding or reducing skin irritation.

Furthermore, the butter(s) and the oil(s) may act as refatting ingredients, which may moisturize irritated skin and may help the skin regenerate. In addition, the butter(s) and oil(s) may facilitate formation of a glide film for the razor cartridge to glide on during the shaving process, again reducing the skin irritation.

Accordingly, in a first aspect, the present disclosure relates to a razor cartridge comprising a housing having a front edge and a rear edge. The housing comprises one or more shaving blades disposed between the front edge and the rear edge. At least one shaving bar is disposed in front of the blade(s) and/or rear of the blade(s). The shaving bar comprises a solid shaving aid composition. The solid shaving aid composition may comprise, relative to the weight of the solid shaving aid composition: about 25 wt.-% to about 75 wt.-% of a soap base consisting of one or more fatty acid salts having from about 8 to about 24 carbon atoms and optionally one or more fatty acids having from about 8 to about 24 carbon atoms, about 15 wt.-% to about 60 wt.-% of one or more polyols comprising from about 2 to about 8 carbon atoms, about 0.1 wt.-% to about 3.0 wt.-% of one or more non-ionic surfactants, about 0.1 wt.-% to about 3.0 wt.-% of one or more zwitterionic surfactant, one or more butters, and one or more oils.

The shaving bar is not particularly limited and can have any suitable shape. Typically, it is in the form of at least one strip which is arranged substantially parallel to the shaving blades. The shaving bar comprises the shaving aid composition. The shaving aid composition is solid for the present purposes, i.e. solid in the sense that the shaving aid composition retains its shape once applied to the cartridge and does not migrate or "run off" under normal storage conditions (e.g. temperature of up to about 30°C and a relative humidity of up to about 60%). However, it should be understood that that the solid state of the shaving aid composition is not particularly limited and also includes wax-like characteristics. The shaving aid composition may be solid at room temperature, e.g. at about 25°C.

In some embodiments the solid shaving aid composition may comprise one or more fatty acid salts or fatty acids comprising from about 8 to about 24 carbon atoms. The term fatty acid is well-established in this technical filed and is in particular meant to include a monovalent linear carboxylic acid having between about 8 and about 24 carbon atoms.

When referring to the soap base in the present disclosure, it should be understood that this is referring to the sum of the aforementioned fatty acid salts and fatty acids as a (major) component of the soap solid shaving aid. Additionally or alternatively, the soap base may be defined as the total summed-up amount of the aforementioned fatty acid salts and, if present, fatty acids.

In some embodiments, the one or more fatty acid salts may comprise one or more monovalent linear or branched saturated or unsaturated carboxylic acid salts having between about 8 and about 24 carbon atoms, in particular between about 10 and about 20 carbon atoms. In some embodiments, the solid shaving aid composition may optionally comprise one or more monovalent linear or branched saturated or unsaturated carboxylic acids having between about 8 and about 24 carbon atoms, in particular between about 10 and about 20 carbon atoms. The fatty acid salts may be partly substituted by said fatty acids, e.g. from about 0.1 to about 20 wt-%, in particular from about 1 to about 10 wt.-%, relative to the total weight of the fatty acid salts. In some embodiments, the solid shaving aid composition may be substantially free or free of fatty acids.

In some embodiments, the one or more fatty acid salts may comprise one or more salts of cocoate, stearate, palmitate, laurate, myristate, oleate, acyl sarcocinate, or mixtures thereof.

In some embodiments the solid shaving aid composition may comprise the one or more fatty acid salts and the optional one or more fatty acids in a total amount of between about 25 to about 70 wt.-%, and in particular between about 28 wt.-% and about 65 wt.-%, relative to the total weight of the solid shaving aid composition.

The fatty acids and fatty acid salts may regulate the consistency of the solid shaving aid. Furthermore, the fatty acids and fatty acid salts may further act as surfactant and provide emulsion stabilization to the solid shaving aid. In this and other contexts, it should be noted that the present disclosure does not intend to distinguish between tensides, surfactants, and emulsifiers. All these compounds are endowed with a certain degree of surface-active activity and are used interchangeably unless expressly stated otherwise.

The one or more polyols may comprise from about 2 to about 8 carbon atoms. Suitable examples include polyols comprising from about 2 to about 8 carbon atoms and from about 2 and about 7, more specifically from about 2 and about 6, hydroxyl groups. Mixtures of several of such compounds may also be comprised. Suitable polyols may particular be selected from the group consisting of glycerol, sorbitol, ethylene glycol, diethylene glycol, propylene glycol, dipropylene glycol, tripropylene glycol, methylpropane diol glycol, and mixtures thereof.

In some embodiments the solid shaving aid composition may comprise the one or more polyols in an amount of between about 15 wt.-% to about 55 wt.-% and in particular between about 17 wt.-% and about 52 wt.-%, relative to the total weight of the solid shaving aid composition.

The one or more polyols may act as moisturizers. Additionally, the one or more polyols may act as an (additional) solvent, which may facilitate production of the solid shaving aid.

Examples of polyols include glycerol and sorbitol. Examples of solvents include propylene glycol, ethylene glycol, diethylene glycol, dipropylene glycol, tripropylene glycol, and methylpropane diol glycol.

In some embodiments, the solid shaving aid composition may comprise a relatively large amount of lower-molecular-weight polyols which may be better able to penetrate into the skin and hair and which may have a lower tendency accumulate on the skin, potentially forming a (sticky) film on the surface of the skin. Such lower-molecular-weight polyols may i.a. be characterized by a lower melting point. Accordingly, in some embodiments, the solid shaving aid composition may comprise at least about 10 wt.-%, in particular at least about 12 wt.-%, of polyols having a melting point below about 34°C. In some embodiments, the solid shaving aid composition may comprise at least about 10 wt.-%, in particular at least about 12 wt.-%, of polyols selected from the group consisting of glycerol, ethylene glycol, diethylene glycol, propylene glycol, dipropylene glycol, tripropylene glycol and methylpropane diol glycol. In some embodiments, the solid shaving aid composition may comprise at most about 25 wt.-%, in particular at most about 22wt.-%, sorbitol. All of the aforementioned weight-references arerelative to the total weight of the solid shaving aid composition.

In some embodiments, the solid shaving aid composition may comprise, as the one or more polyols, relative to the total weight of the solid shaving aid composition:
about 5 to about 30 wt.-%, in particular about 7 to about 25 wt.-%, glycerol;
about 5 to about 30 wt.-%, in particular about 7 to about 25 wt.-%, sorbitol; and
about 2 to about 15 wt.-%, in particular about 3 to about 10 wt.-%, of a compound selected from propylene glycol, ethylene glycol, dipropylene glycol, tripropylene glycol, methylpropane diol glycol, and mixtures thereof, in particular propylene glycol.

In some embodiments the solid shaving aid composition may comprise, relative to the total weight of the solid shaving aid composition, between about 0.2 wt.-% to about 2.5 wt.-% and in particular between about 0.3 wt.-% to about 2.0 wt.-%, of the non-ionic surfactant.

The nonionic surfactants may, for example, be chosen from those compounds that are well known per se (see, for example, "Handbook of Surfactants" by M. R. Porter, published by Blackie & Son (Glasgow and London), 1991, pp. 116-178) which are incorporated herein by reference. In some embodiments the non-ionic surfactant may be selected from polyethoxylated, polypropoxylated and polyglycerolated fatty acids; alkylphenols; copolymers of ethylene oxide and of propylene oxide; condensates of ethylene oxide and of propylene oxide with fatty alcohols; polyethoxylated fatty amides; polyglycerolated fatty amides; polyethoxylated fatty amines; oxyethylenated fatty acid esters of sorbitan; fatty acid esters of sucrose; fatty acid esters of polyethylene glycol; and alkylpolyglycosides.

In some embodiments, the solid shaving aid may comprise α-diols and alcohols comprising at least one fatty chain comprising, for example, from about 8 to about 18 carbon atoms, it being possible for the number of ethylene oxide or propylene oxide groups to range, for example, from about 2 to about 200 and for the number of glycerol groups to range, for example, from about 2 to about 100.

In some embodiments, the solid shaving aid may comprises polyethoxylated, polypropoxylated and polyglycerolated C₈-C₃₀ fatty acid with a polymerization degree of 2 to about 200, more specifically a polyethylene glycolester of C₈-C₃₀ fatty acid, and in particular a polyethylene glycolester of stearic acid with a polymerization degree of 2 to about 200, such as PEG-100 stearate.

The non-ionic surfactants may also be selected from copolymers of ethylene oxide and of propylene oxide, condensates of ethylene oxide and of propylene oxide with C₈-C₃₀-fatty alcohols; polyethoxylated C₈-C₃₀-fatty amines, for example, those comprising from about 2 to about 30 mol of ethylene oxide, polyglycerolated C₈-C₃₀-fatty amides comprising on average from about 1 to about 5 glycerol groups, for example, from about 1.5 to about 4, glycerol groups; polyethoxylated C₈-C₃₀-fatty amines, for example, comprising from 2 to 30 mol of ethylene oxide; oxyethylenated C₈-C₃₀-fatty acid esters of sorbitan comprising from 2 to 30 mol of ethylene oxide; C₈-C₃₀-fatty acid esters of sucrose, C₈-C₃₀-fatty acid esters of polyethylene glycol, C₈-C₃₀-alkylpolyglycosides, and N-C₈-C₃₀-alkylglucamine derivatives.

The non-ionic surfactants may also be selected from alkylpolyglycosides, in particular C₈-C₃₀-alkylpolyglycosides.

In some embodiments, the non-ionic surfactant may be glycerol monoesterified with a fatty acid as defined above, in particular glyceryl laurate.

Non-ionic surfactants may act a mild cleansing agents and emollients and may reduce skin irritation.

In some embodiments the at least one zwitterionic surfactant may be present in amount of about 0.2 wt.-% to about 2.5 wt.-%, and in particular in amounts of about 0.3 wt.-% to about 2.0 wt.-%, relative to the total weight of the solid shaving composition.

In some embodiments the at least one zwitterionic surfactant may comprise a positively charged nitrogen-based group, in particular a quaternary ammonium group, and a negatively charged group, in particular a sulfonate or carboxylate group.

The zwitterionic surfactants can be, for example, selected from aliphatic secondary and tertiary amine derivatives wherein the aliphatic radical is selected from linear and branched chains comprising from about 8 to about 22 carbon atoms and comprising at least one water-soluble anionic group, for example, at least one water-soluble anionic group selected from carboxylate, sulfonate, sulfate, phosphate and phosphonate groups.

In some embodiments the at least one zwitterionic surfactant may comprise a betaine and/or sulfobetaines (sultaines).

In some embodiments the at least one zwitterionic surfactant may comprise a betaine selected from (C₈-C₂₀)alkylbetaines, sulfobetaines, (C₈-C₂₀)alkylamido(C₁-C₆)alkylbetaines; and (C₈-C₁₀)alkylamido(C₁-C₆)alkylsulfobetaines which may optionally be oxyethylenated.

In some embodiments the at least one zwitterionic surfactant may comprise cocoamidopropylbetaine.

In some embodiments the at least one zwitterionic surfactant may be pH-insensitive. pH-insensitivity may refer to the surfactant not changing its ionic charge of its cationic functional groups when the pH is changed within usual physiological ranges (about pH 3.5 to about pH = 9). For instance, quaternary ammonium-based surfactants may be pH-insensitive whereas ammonium-based tensides are pH-sensitive since the protonated amino group will change its degree of protonation in the pH-range of about 3.5 to about 9.

The presence of zwitterionic surfactant may provide further mild cleansing power. Furthermore, zwitterionic surfactants may also act as skin conditioners and may reduce skin irritations.

In some embodiments the solid shaving aid composition may comprise water, in particular in an amount between about 2.0 wt.-% to about 35 wt.-%, in particular about 5.0 wt.-% to about 25 wt.-%, relative to the total weight of the solid shaving aid composition.

In some embodiments the total content of butter(s) may be between 0.5 wt.-% to 5.0 wt.-%, in particular about 0.5 % to about 4.0 wt.-%, relative to the total weight of the solid shaving aid composition.

For the purpose of the present invention, the term "butter" is intended to mean a lipophilic fatty compound or composition with a reversible solid/liquid change of state, comprising, in its pure form and at a temperature of about 25°C, a liquid and a solid fraction. In other words, the starting melting point of the butter can be less than about 25°C. The liquid fraction of the butter measured at about 25°C can represent between about 9 wt.-% and about 97 wt.-% of the compound, more specifically about 15 wt.-% and about 85 wt.-%, and in particular between about 40 wt.-% and about 85 wt.-%.

In some embodiments, the butter(s) may have an end melting point of less than about 60°C.

For the purposes of this disclosure, the melting point of a butter may correspond to the temperature of the most endothermic peak observed on thermal analysis (DSC) as described in Standard ISO 1 1357-3:1999. Said melting point may e.g. be measured using a differential scanning calorimeter (DSC), for example the calorimeter sold under the name DSC Q2000 by the company TA Instruments, USA. As regards the measurement of the melting point and the determination of the end melting point, an exemplary sample preparation and measurement protocols may be as follows:

A sample of 5 mg of the butter, preheated to 80°C and withdrawn with magnetic stirring using a spatula that is also heated, is placed in a hermetic aluminium capsule, or crucible. Two tests are performed to ensure the reproducibility of the results. The measurements are performed on the abovementioned calorimeter. The oven is flushed with nitrogen. Cooling is provided by an RCS 90 heat exchanger. The sample is then subjected to the following protocol: it is first of all placed at a temperature of 20°C, and then subjected to a first temperature rise passing from 20°C to 80°C, at a heating rate of 5°C/minute, then is cooled from 80°C to -80°C at a cooling rate of 5°C/minute and finally subjected to a second temperature rise passing from -80°C to 80°C at a heating rate of 5°C/minute. During the second temperature rise, the variation in the difference in power absorbed by the empty crucible and by the crucible containing the sample of butter is measured as a function of the temperature. The melting point of the butter is the value of the temperature corresponding to the top of the peak of the curve representing the variation in the difference in power absorbed as a function of the temperature.

The end melting point corresponds to the temperature at which 95% of the sample has melted.

The liquid fraction by weight of the butter at 25°C is equal to the ratio of the heat of fusion consumed at 25 °C to the heat of fusion of the butter.

The heat of fusion of the butter is the heat consumed in order to transition from the solid state to the liquid state. The heat of fusion of the butter is equal to the integral of the entire melting curve obtained using the abovementioned calorimeter, with a temperature rise of 5 or 10°C/minute, according to Standard ISO 1 1357-3:1999. The heat of fusion of the butter is the amount of energy required to make the butter change from the solid state to the liquid state. It is expressed in J/g.

The heat of fusion consumed at 25°C is the amount of energy absorbed by the sample to change from the solid state to the state that it has at 25°C, consisting of a liquid fraction and a solid fraction.

The liquid fraction of the butter measured at 32°C may represent from 30% to 100% by weight of the butter, more specifically from 50% to 100%, and in particular from 60% to 100% by weight of the compound. When the liquid fraction of the butter measured at 32°C is equal to 100%, the temperature of the end of the melting range of the butter is less than or equal to 32°C.

The liquid fraction of the butter measured at 32°C is equal to the ratio of the heat of fusion consumed at 32°C to the heat of fusion of the butter. The heat of fusion consumed at 32°C is calculated in the same way as the heat of fusion consumed at 23°C.

In some embodiments, the butter may have a hardness of less than or equal to 6 about MPa. As regards the measurement of the butter hardness, an exemplary sample preparation and measurement protocols may be as follows:
The butter is placed in a mould 75 mm in diameter, which is filled to about 75% of its height. In order to overcome the thermal history and to control the crystallization, the mould is placed in a Votsch VC0018 programmable oven, where it is first of all placed at a temperature of 80°C for 60 minutes, then cooled from 80°C to 0°C at a cooling rate of 5°C/minute, then left at the stabilized temperature of 0°C for 60 minutes, and then subjected to a temperature rise passing from 0°C to 20°C, at a heating rate of 5°C/minute, and then left at the stabilized temperature of 20°C for 180 minutes.

The compressive force measurement is taken using a TA/TX2i texturometer from Swantech. The spindle used is chosen according to the texture:
- cylindrical steel spindle 2 mm in diameter for very rigid starting materials;
- cylindrical steel spindle 12 mm in diameter for relatively non-rigid starting materials.

The measurement comprises three steps:
- a first step after automatic detection of the surface of the sample, where the spindle moves at a measuring speed of 0.1 mm/second, and penetrates into the composition according to the invention or the butter to a penetration depth of 0.3 mm, and the software notes the maximum force value reached;
- a second step, known as relaxation, where the spindle remains in this position for one second and where the force is noted after 1 second of relaxation; and finally
- a third step, known as withdrawal, where the spindle returns to its original position at a speed of 1 mm/second, and the withdrawal energy of the probe (negative force) is noted.

The hardness value measured during the first step corresponds to the maximum compressive force measured in newtons divided by the area of the texturometer cylinder expressed in mm² in contact with the butter or the composition according to the invention. The hardness value obtained is expressed in megapascals or MPa.

Additionally or alternatively, a butter may be characterized in the solid state (at about 25°C) by an anisotropic crystal organization, which is visible by X-ray observation. Methods for determining the presence of anisotropic crystal organization within butters are not particularly limited and include XRD and in particular X-ray birefringence imaging (XBI).

In some embodiments, the butter(s) is (are) of plant origin, in particular such as those described in Ullmann's Encyclopedia of Industrial Chemistry ("Fats and Fatty Oils", A. Thomas, Published Online: 15 JUN 2000, DOI: 10.1002/14356007.a10_173, point 13.2.2.2. Shea Butter, Borneo Tallow, and Related Fats (Vegetable Butters)) which are incorporated herein by reference.

In some embodiments, the butter(s) comprise butters selected form the following list: Shea butter, Nilotica shea butter (Butyrospermum parkii), galam butter (Butyrospermum parkii), Borneo butter or fat or tengkawang tallow (Shorea stenoptera), shorea butter, illipe butter, madhuca butter or (Bassia) Madhuca longifolia butter, mowrah butter (Madhuca latifolia), katiau butter (Madhuca mottleyana), phulwara butter (M. butyracea), mango butter (Mangifera indica), murumuru butter (Astrocaryum murumuru), kokum butter (Garcinia indica), ucuuba butter (Virola sebifera), tucuma butter, painya (kpangnan) butter (Pentadesma butyracea), coffee butter (Coffea arabica), apricot butter (Prunus armeniaca), macadamia butter (Macadamia ternifolia), grapeseed butter (Vitis vinifera), avocado butter (Persea gratissima), olive butter (Olea europaea), sweet almond butter (Prunus amygdalus dulcis), cocoa butter (Theobroma cacao) and sunflower butter.

In some embodiments the at least one butter may be selected from the group consisting of cocoa butter, mango butter, shea butter and mixtures thereof.

In some embodiments the solid shaving aid composition comprises cocoa butter, mango butter, and shea butter.

Butters may help in moisturizing dry and irritated skin. Further, butters may provide skimsoothing and regenerative properties. Furthermore, when in contact with warm skin, butters may melt and provide a film on the skin, which may provide improved glide and maneuverability to the razor.

The solid shaving aid composition may comprise an oil.

Within the present disclosure, the term "oil" refers to C₈-C₅₀-substances that are liquid at room temperature (about 25°C). These oils may be hydrocarbon-based oils of animal, plant, mineral or synthetic origin, silicone oils and/or fluoro oils, and mixtures thereof. The term "hydrocarbon-based oil" refers to an oil mainly containing carbon atoms and hydrogen atoms (e.g. at least about 70 or about 80 atomic wt.-%) .

Specific examples of oils that can be used include:
- hydrocarbon-based oils of animal origin, such as perhydrosqualene (squalane);
- hydrocarbon-based plant oils, such as liquid triglycerides of fatty acids of 4 to 10 carbon atoms, such as heptanoic or octanoic acid triglyceride; coconut oil, sunflower oil, corn oil, soybean oil, marrow oil, grapeseed oil, sesame oil, hazelnut oil, apricot oil, macadamia oil, arara oil, castor oil, avocado oil, caprylic/capric acid triglycerides, and jojoba oil;
- linear or branched hydrocarbons of mineral or synthetic origin, such as liquid paraffin and derivatives thereof, petroleum jelly, polydecenes and hydrogenated polyisobutene, such as parleam;
- synthetic esters and ethers, in particular, of fatty acids, such as the oils of formula R₁₅COOR₁₆ in which R₁₅ represents a fatty acid residue containing from about 7 to about 25 carbon atoms and R₁₆ represents a hydrocarbon-based chain containing from about 3 to about 25 carbon atoms, such as, for example, purcellin oil, isononyl isononanoate, isopropyl myristate, 2-ethylhexyl palmitate, 2-octyldodecyl stearate, 2-octyldodecyl erucate, isostearyl isostearate; hydroxylated esters such as isostearyl lactate, octyl hydroxystearate, octyldodecyl hydroxystearate, diisostearyl malate, triisocetyl citrate, fatty alkyl heptanoates, octanoates or decanoates; polyol esters such as propylene glycol dioctanoate, neopentyl glycol diheptanoate, diethylene glycol diisononanoate; and pentaerythritol esters such as pentaerythrityl tetraisostearate;

In some embodiments the one or more oils may comprise an oil selected chosen from triglycerides and/or triglyceride derivatives and in particular a coconut oil.

In some embodiments, it may be advantageous that the one or more oils comprises a glycerol esterified with three fatty acids. The fatty acids may be as defined above.

In some embodiments, the solid shaving aid composition may comprise, relative to the total weight of the solid shaving aid composition, between about 0.1 wt.-% to about 4 wt.-%, more specifically between about 0.4 wt.-% and about 3 wt.-%, and in particular between about 0.6 wt.-% and about 2.0 wt.-%, of the one or more oils.

Oils may be added to provide a film on the skin, which may improve the glide and maneuverability of the razor. It may be particularly advantageous that the oils comprise a squalene and/or a coconut oil.

In some embodiments, the aforementioned one or more oils may comprise one or more humectant oils. The humectant oils may have a molecular weight of greater than about 500 g/mol, more specifically greater than about 700 g/mol, and in particular greater than about 900 g/mol. In some embodiments the humectant oils may comprise a castor oil derivative, more specifically a castor oil esterified with 1, 2 or 3 dicarboxylic acid(s) having between about 3 and about 8 carbon atoms. In particular, the humectant oil may be castor oil derivative, and in particular castoryl maleate.

In some embodiments, the solid shaving aid composition may comprise, relative to the total weight of the solid shaving aid composition, between about 0.1 wt.-% to about 2 wt.-%, more specifically between about 0.2 wt.-% and about 1 wt.-%, and in particular between about 0.2 wt.-% and about 0.8 wt.-%, of the aforementioned one or more humectant oils having a molecular weight of greater than about 500 g/mol.

In some embodiments, the solid shaving aid composition may comprise, relative to the total weight of the solid shaving aid composition, between about 0.1 wt.-% to about 2 wt.-%, more specifically between about 0.2 wt.-% and about 1 wt.-%, and in particular between about 0.3 wt.-% and about 0.8 wt.-%, of squalane.

In some embodiments, the solid shaving aid composition may comprise, relative to the total weight of the solid shaving aid composition, about 0.1 wt.-% to about 2 wt.-%, more specifically between about 0.2 wt.-% and about 1 wt.-%, and in particular between about 0.2 wt.-% and about 0.8 wt.-%, of the one or more humectant oils having a molecular weight of greater than about 500 g/mol; between about 0.1 wt.-% to about 2 wt.-%, more specifically between about 0.2 wt.-% and about 1 wt.-%, and in particular between about 0.3 wt.-% and about 0.8 wt.-%, of squalane; and, in total, between about 0.3 wt.-% to about 6 wt.-%, more specifically between about 0.4 wt.-% and about 4 wt.-%, and in particular between about 0.5 wt.-% and about 4 wt.-%, of the one or more oils.

The humectant oil may improve skin hydration. Further, some humectants oil such as castoryl maleate and its derivatives may contribute to providing a film on the skin which may improve gliding and maneuverability of a razor cartridge. Squalane may further act as emollient and improve gliding and maneuverability of a razor cartridge. The combination of humectant oil and squalane may be particularly well-suited to improve both skin hydration and gliding and maneuverability of a razor cartridge.

In some embodiments the solid shaving aid composition may comprise one or more silicone cross-polymers.

In some embodiments the one or more silicone cross-polymers may comprise an emulsifying silicone cross-polymer.

In some embodiments the one or more silicone cross-polymers may comprise a first (di)methicone-based polymer chain which is crosslinked to a second (di)methicone-based polymer chain via at least one hydrophilic oligomer/polymer chain.

In some embodiments the first and/or second dimethicone-based polymer chain may be selected from dimethicone and optionally substituted linear or branched saturated or unsaturated C₁-C₂₀ alkyl (di)methicone.

In some embodiments the hydrophilic oligomer/polymer chain may be a C₁-C₄-alkyl polyether wherein the C₁-C₃-alkyl residues are optionally substituted with hydroxyl, hydroxyl-C₁-C₄-alkyl, C₁-C₄-alkyl ether groups or hydroxyl-C₁-C₄-alkylether groups, in particular polyethylene glycol, polypropylene glycol, polyglycerol, and polysorbate.

In some embodiments the one or more silicone cross-polymer may comprise polyglycerine-modified cross-linked polymers, polyether-modified cross-linked polymers, polyether/alkyl co-modified cross-linked polymers, polyglycerine/alkyl co-modified cross-linked polymers, dimethicone/vinyl dimethicone cross-polymers, dimethicone/phenyl vinyl dimethicone crosspolymers, vinyl dimethicone/lauryl dimethicone cross-polymers, dimethicone crosspolymers, C₃₀₋₄₅- alkyl cetearyl dimethicone cross-polymers, cetearyl dimethicone cross-polymers, in particular dimethicone/vinyl dimethicone cross-polymers, vinyl dimethicone/lauryl dimethicone cross-polymers, vinyl dimethicone/lauryl dimethicone cross-polymers, lauryl dimethicone/polyglycerin cross-polymers, dimethicone/polyglycerin cross-polymers, polyethylene glycol/polypropoylene glycol-dimethicone cross-polymer; and mixtures thereof.

In some embodiments the one or more silicone cross-polymers may be present in amounts of about 0.1 to about 1.0 wt.-%, more specifically between about 0.15 to about 0.7 wt.-%, and in particular between about 0.20 wt.-% and about 0.45 wt.-%, relative to the total weight of the solid shaving aid composition.

In some embodiments, the solid shaving aid composition may further comprise an anionic surfactant selected from sulfur-containing anionic surfactants, in particular anionic surfactants selected from alkyl sulfates, sulfosuccinates, alkyl benzene sulfanate, acyl methyl taurates, isethionates, monoglyceride sulfates and ether sulfanates having between about 8 and about 24 carbon atoms, in particular between about 10 and about 20 carbon atoms. However, in view of their skin irritating potential and such surfactants to amounts, relative to the total weight of the solid shaving aid composition, of less than about 3.0 wt.-%, such as between about 0.1 wt.-% and about 2.0 wt.-%, or between about 0.2 wt.-% and about 1.5 wt.-%.

In some embodiments the solid shaving aid composition may further comprise between about 2.0 wt.-% and about 8.0 wt.-%, and in particular between 4.0 wt.-% and about 6.5 wt.-% of one or more bulking agents, relative to the total weight of the solid shaving aid composition.

In some embodiments, the bulking agent may comprise sodium chloride.

In some embodiments the solid shaving aid composition may further comprise an number of generally minor optional ingredients which will be discussed below. It should be understood that the below list is not exhaustive and does not exclude the presence of other ingredients not listed below.

In some embodiments the solid shaving aid composition may comprise between about 0.04 wt.-% and about 5 wt.-%, and in particular between about 1.5 wt.-% and about 3.5 wt.-% of one or more opacifying agents, relative to the total weight of the solid shaving aid composition.

In some embodiments the opacifying agent may comprise an inorganic pigment, in particular titanium dioxide, and/or a styrene/acrylate copolymer.

In some embodiments the solid shaving aid composition may comprise one or more chelating agents.

In some embodiments the chelating agent may comprise a (multidentate) chelating agent, in particular pentasodium pentetate, tetrasodium etidronate, tetrasodium iminodissucinate, citric acid, sodium citrate or mixtures thereof.

In some embodiments the solid shaving aid composition may comprise between about 0.01 wt.-% and about 2 wt.-%, and in particular between about 0.08 wt.-% and about 1.5 wt.-% of one or more multidentate chelating agents, relative to the total weight of the solid shaving aid composition.

In some embodiments the solid shaving aid composition further may comprise, relative to the total weight of the solid shaving aid composition, between about 0.1 wt.-% and about 2.5 wt.-%, in particular between about 0.3 wt.-% and about 1.5 wt.-%, of a fragrance or mixture of fragrances.

In some embodiments the solid shaving aid composition may further comprise, relative to the total weight of the solid shaving aid composition, between about 0.01 wt.-% and about 2.0 wt.-%, in particular between about 0.05 wt.-% and about 1.0 wt.-%, of an anti-oxidant or mixture of anti-oxidants.

In some embodiments the solid shaving aid composition may be substantially free or free of silicone oil.

In some embodiments, the solid shaving aid composition may comprise a minor amount of waxes, for instance an amount of 0.1 to about 6 wt.-%, more specifically between about 0.5 and about 3 wt.-%, relative to the total weight of the solid shaving aid composition. In some embodiments, the solid shaving aid composition may comprise less than about 6 wt.-% or less than about 3 wt.-% of a wax, relative to the total weight of the solid shaving aid composition. In some embodiments the solid shaving aid composition may be substantially free or free of waxes.

The term "wax," in the present disclosure is intended to be used as is well-established in the field of cosmetics and is, in particular, meant to refer to a lipophilic (fatty) compound that is solid at e.g. room temperature (e.g. about 25 °C) with a reversible solid/liquid change of state. The method of measuring the melting point of the wax is not particularly limited and may be measured using a differential scanning calorimeter (DSC), for example the calorimeter sold under the name DSC 3 by the company Mettler Toledo.

In some embodiments, the one or more waxes may have a melting point of at least about 40°C. A higher melting point of the one or waxes may facilitate the aforementioned film-forming capabilities of the composition and may facilitate retaining the effect on the warm skin of the user or when in contact to warm water. In some embodiments, the wax may have a melting point of at least about 40°C, more specifically at least about 42°C and in particular at least about 45°C. In some embodiments, the wax may have a melting point range of between about 40°C and about 120°C, more specifically between about 42°C and about 100°C, and in particular between about 45°C and about 80°C.

In some embodiments, the wax may have a solubility in water at about 25°C of less than about 1000 mg/L, more specifically a solubility of less than 100 mg/L or less than about 10 mg/L, and in particular a solubility of less than about 1 mg/L. In some embodiments, the wax may be substantially insoluble or insoluble in water at about 25 °C. The method of determining the solubility of the wax is not particularly limited and may be performed by any suitable means, for instance using a USP Dissolution Apparatus 2 (paddle type). A lower water solubility may also facilitate the aforementioned film-forming capabilities of the composition.

Examples of waxes include the following:

### a) Hydrocarbon-based wax

In some embodiments, the wax may be a hydrocarbon-based wax. The term "hydrocarbon-based wax" is intended to refer to a wax formed essentially from, or even constituted by, carbon and hydrogen atoms, and possibly oxygen and nitrogen atoms, and not containing any silicon or fluorine atoms. It may contain functional groups such as alcohol, ether, amine and/or amide groups.

In some embodiments one or more of the waxes may comprise a hydrocarbon-based wax, in particular a paraffin.

In some embodiments one or more of the waxes may comprise a hydroxylated hydrocarbon-based wax. In some embodiments, in particular in cases where the hydrocarbon-based wax is a co-surfactant, it may be particularly advantageous that the hydrocarbon-based wax comprises a linear or branched, saturated or unsaturated, monovalent or multivalent hydrocarbyl alcohol having between about 8 and about 32carbon atoms, specifically between about 10 and about 28 carbon atoms, and in particular between about 12 about 24 carbon atoms. More specifically, the wax may comprise an (in particular a linear) mono- or divalent alkyl alcohol having between about 8 and about 32 carbon atoms, specifically between about 10 and about 28 carbon atoms, and in particular between about 12 about 24 carbon atoms. In some embodiments, the wax may comprise a mono- or divalent fatty alcohol, in particular cetyl alcohol, stearyl alcohol, behenyl alcohol, or mixtures thereof such as cetearyl alcohol. A commercial example includes Lanette O, sold by BASF SE, Germany. A fatty alcohol is the aforementioned sense may in particular be a monovalent linear alkyl alcohol having between about 8 and about 24 carbon atoms.

### b) Ester wax

In some embodiments the wax may comprise an ester wax, in particular a hydrocarbon-based ester wax. The term "ester wax" in the present disclosure is intended to be used as is well-established in the field of cosmetics and is, in particular, meant to refer to a wax comprising at least one ester group.

In some embodiments, the ester wax is an ester of a C₈-₄₀-hydrocarbyl carboxylic acid, which may optionally be substituted with one or more heteroatoms such as O, N, S or P, with a C₂-C₈ polyol having between 2 and 8 hydroxyl groups. In some embodiments, the C₈-C₄₀-hydrocarbyl carboxylic acid may comprise at least one saturated or unsaturated C₁₀-C₃₂-moiety, more specifically at least one saturated or unsaturated C₁₂-C₂₆-moiety, and in particular at least one saturated or unsaturated C₁₄-C₂₄-moiety. In some embodiments, the C₂-C₈ polyol has between 2 and 6, between 2 and 4, between 3 and 6, or between 3 and 5 hydroxyl groups. In some embodiments, the ester wax is a monoester, a diester, a triester or a tetraester, and in particular a monoester or diester.

In some embodiments, it may be advantageous that the ester wax still comprises hydroxyl groups, i.e. that the ester is (formally) formed by reaction of less C₈-C₄₀-hydrocarbyl carboxylic acids than the number of hydroxyl groups in the C₂-C₈ polyol and/or that the C₈-C₄₀-hydrocarbyl carboxylic acids are substituted with hydroxyl groups. In some embodiments, it may thus be advantageous that the ester wax comprises one, two, three or four (free) hydroxyl groups. The remaining hydroxyl groups may have the advantage of providing co-surfactant performance to the ester wax.

In some embodiments, the ester wax is an ester of a C₈-C₄₀-hydrocarbyl alcohol, which may optionally be substituted with one or more heteroatoms such as O, N, S or P, with a C₂-C₈ carboxylic acid having between 2 and 6 carboxylic acid groups. In some embodiments, the C₈-C₄₀-hydrocarbyl alcohol may comprise at least one saturated or unsaturated C₁₀-C₃₂-moiety, more specifically at least one saturated or unsaturated C₁₂-C₂₆-moiety, and in particular at least one saturated or unsaturated C₁₄-C₂₄-moiety. In some embodiments, the C₂-C₈ carboxylic acid has between 2 and 6, between 2 and 4, between 3 and 6, or between 3 and 5 carboxylic acid groups. In some embodiments, the ester wax is a mono ester, a diester, a triester or a tetraester.

In some embodiments, it may be advantageous that the ester wax still comprises hydroxyl groups, i.e. that the ester is (formally) formed by reaction of more C₈-C₄₀-hydrocarbyl alcohols than the number of carboxylic acid groups in the C₂-C₈ carboxylic acid and/or that the C₈-C₄₀-hydrocarbyl alcohols are substituted with hydroxyl groups. In some embodiments, it may thus be advantageous that the ester wax comprises one, two, three or four (free) hydroxyl groups. The remaining hydroxyl groups may have the advantage of providing co-surfactant performance to the ester wax.

In some embodiments, it may be advantageous that the ester wax comprises an ester of a fatty alcohol and/or an ester of a polyol. In some embodiments, the fatty alcohol is as defined above or, alternatively, a C₈-C₄₀-hydrocarbyl alcohol. In some embodiments, the polyol is a C₂-C₈ polyol, more specifically glycol or glycerol and in particular glycerol. In both cases, the carboxy-group may be formed by a fatty acid. A fatty acid is this sense may in particular be a monovalent linear carboxylic acid having between about 8 and about 24 carbon atoms.

Non-limiting examples of ester waxes include:
i) beeswax which is a mixture of ester waxes comprising as its main constituents palmitate, palmitoleate, and oleate esters of long-chain (30-32 carbons) aliphatic alcohols, with the ratio of triacontanyl palmitate CH₃(CH₂)₂₉O-CO-(CH₂)₁₄CH₃ to cerotic acid CH₃(CH₂)₂₄COOH, the two principal constituents, being about 6:1.
ii) carnauba wax.
iii) mono- and di-C₈-C₄₀-hydrocarbyl esters of glycol or glycerol such as glyceryl stearate, glyceryl distearate, glycol stearate and glycol distearate. Such ester waxes are available from SABO S.p.A, Italy.
iv) C₂₀-C₄₀ alkyl (hydroxystearyloxy)stearates. Such waxes are sold, for example, under the trade names "Kester Wax K 82 P", "Hydroxypolyester K 82 P", "Kester Wax K 80 P", or "Kester Wax K82H" by the company Koster Keunen BV, Netherlands.
v) esters of ethylene glycol and montanic acid (octacosanoic acid). Such waxes are sold, for example, under the trade name "Licowax KPS" by the company Clariant AG, Switzerland.
vi) bis(1,1,1-trimethylolpropane) tetrastearate. Such a wax is sold under the name Hest 2T-4S^{®} by the company Heterene Chemical Co, Inc., USA.
vii) waxes obtained by hydrogenation of castor oil esterified with cetyl alcohol. Such waxes are sold under the trade names Phytowax ricin 16L64^{®} and 22L73^{®} by the company Sophim SA, France.

In some embodiments, the wax may be a polar wax. For the purposes of the present disclosure, the term "polar wax" denotes a wax comprising at least one polar group. Polar groups are well known to those skilled in the art: they may *i.a.* be selected from hydroxyl, ester, carboxylic acid or amide groups. Such polar waxes are to be differentiated from apolar waxes which are hydrocarbon-based and are substantially free or free of the aforementioned polar groups.

Examples of polar waxes include the aforementioned linear or branched, saturated or unsaturated, monovalent or multivalent hydrocarbyl alcohol having between about 8 and about 32 carbon atoms and the aforementioned ester waxes.

In some embodiments, the wax may be an apolar wax. Examples of such apolar waxes include polyethylene waxes, microcrystalline waxes, ozokerine and Fisher-Tropsch waxes.

In some embodiments, the solid shaving aid composition may comprise the following components wherein the components refer to the components as defined above:
a) about 25 wt.-% to about 75 wt.-% of a soap base consisting of one or more fatty acid salts having from about 8 to about 24 carbon atoms and optionally one or more fatty acids having from about 8 to about 24 carbon atoms,
b) about 15 wt.-% to about 60 wt.-% of one or more polyols comprising from about 2 to about 8 carbon atoms,
c) about 0.1 wt.-% to about 3.0 wt.-% of one or more non-ionic surfactants,
d) about 0.1 wt.-% to about 3.0 wt.-% of one or more zwitterionic surfactants,
e) about 0.5 wt.-% to 5.0 wt.-% of one or more butters, and
f) about 0.1 wt.-% to about 4.0 wt.-% of one or more oils, further optionally including about 0.1 wt.-% to about 2.0 wt.-%, of one or more humectant oils,
g) optionally about 0.1 wt.-% to about 1.0 wt.-%, of one or more silicone cross-polymers,
h) optionally less than about 3.0 wt.-%, such as between about 0.1 wt.-% and about 2.0 wt.-%, of one or more sulfur-containing anionic surfactants,
i) optionally about 2.0 wt.-% to about 35 wt.-% water,
j) optionally up to about 15 wt.-%, in particular about 0.5 to about 10 wt.-%, of further ingredients, in particular the further ingredients defined above (waxes, monovalent alcohols, silicones, opacifiers, fragrances, chelating agents, anti-oxidants, preservatives, etc.) but also other non-mentioned ingredients (e.g. colorants, etc.).

In some embodiments, the solid shaving aid composition may comprise two or three of components g) to j). In some embodiments, the solid shaving aid composition may comprise all components g) to j). In some embodiments, the solid shaving aid composition may consist of components a) to j).

In a second aspect, a solid shaving aid composition as such is provided. The solid shaving aid composition may have a composition as defined in any one of the embodiments or combinations thereof.

In some embodiments the solid shaving aid composition may be configured to last for at least about 8 leg shaves or facial shaves, more specifically at least about 9 leg shaves or facial shaves, and in particular at least about 10 leg shaves or facial shaves.

In some embodiments the solid shaving composition may be shaped into the form of a bar, more specifically a bar configured to be placed onto a razor cartridge, and in particular a bar having a length of between about 30 mm to about 80 mm, a width between about 2 mm and about 10 mm, and a depth of about 0.2 mm to about 4 mm.

The solid shaving aid composition having a composition defined as any combination of the above embodiments, may be used in a multiplicity of facial or leg shaving operations, wherein the facial or leg shaving operations is repeated for at least about 8 times, specifically about 9 times, and in particular at least about 10 times, without replacing the solid shaving aid composition.

In a third aspect, the present disclosure relates to the use of a solid shaving aid composition having a composition defined above in a multiplicity of facial or leg shaving operations, wherein the facial or leg shaving operations is repeated for at least about 8 times, specifically about 9 times, and in particular at least about 10 times, without replacing the solid shaving aid composition.

Specific embodiments according to the third aspect may use any of the solid shaving aid compositions defined for the first aspect of the present disclosure.

### Example

A representative composition according to the below table 1 was prepared by hot mixing the components.

**Table 1: shaving aid composition**

| **Components** | **Amount (wt.-%)** |
|---|---|
| SOAP BASE (SODIUM STEARATE, SODIUM LAURATE, SODIUM OLEATE, SODIUM MYRISTATE) | 30.4 - 62.9 |
| C2-C8 POLYOLS (GLYCERIN, SORBITOL, PROPYLENE GLYCOL, DIPROPYLENE GLYCOL) | 17 - 52 |
| NON-IONIC SURFACTANT (GLYCERYL LAURATE) | 0.2 - 2.0 |
| ZWITTERIONIC SURFACTANT (COCAMIDOPROPYL BETAINE) | 0.2 -2.0 |
| OILS (SQUALANE, CASTORYL MALEATE) | 0.6 - 2 |
| BUTTERS (BUTYROSPERMUM PARKII BUTTER, MANGIFERA INDICA (MANGO) SEED BUTTER, THEOBROMA CACAO (COCOA) SEED BUTTER) | 0.5 - 4.0 |
| SILICONE CROSS-POLYMER (LAURYL DIMETHICONE POLYGLYCERIN-3 CROSSPOLYMER) | 0.1 - 1 |
| AQUA | 11.0 - 23.4 |
| SULFUR-CONTAINING ANIONIC SURFACTANTS (SODIUM LAURYL SULFATE, SODIUM COCOYL ISETHIONATE) | 0.5 - 2 |
| OTHER COMPONENTS | 2 - 10.9 |
| Total | 100 |

The solid shaving aid composition provides good durability and a well-balanced overall shaving performance.

The present disclosure also relates to the following list of embodiments:
1. A solid shaving aid (soap) composition comprising:
   a housing having a front edge and a rear edge;
   one or more shaving blades disposed between the front edge and the rear edge; and
   at least one shaving bar disposed in front of the blade(s) and/or rear of the blade(s) which comprises a solid shaving aid composition;
   wherein the solid shaving aid composition comprises, relative to the total weight of the solid shaving aid composition:
      about 25 wt.-% to about 75 wt.-% of a soap base consisting of one or more fatty acid salts having from about 8 to about 24 carbon atoms and optionally one or more fatty acids having from about 8 to about 24 carbon atoms,
      about 15 wt.-% to about 60 wt.-% of one or more polyols comprising from about 2 to about 8 carbon atoms,
      about 0.1 wt.-% to about 3.0 wt.-% of one or more non-ionic surfactants,
      about 0.1 wt.-% to about 3.0 wt.-% of one or more zwitterionic surfactants,
      one or more butters, and
      one or more oils.
2. The razor cartridge of clause 1, wherein the soap base comprises a monovalent linear or branched saturated or unsaturated carboxylic acid salt having between about 8 and about 24 carbon atoms, in particular between about 10 and about 20 carbon atoms; and/or wherein the soap base comprises a monovalent linear or branched saturated or unsaturated carboxylic acid having between about 8 and about 24 carbon atoms, in particular between about 10 and about 20 carbon atoms.
3. The razor cartridge of clause 1 or clause 2, wherein the soap base comprises one or more salts of cocoate, stearate, palmitate, laurate, oleate and myristate.
4. The razor cartridge of any one of clauses 1 to 3, wherein the solid shaving aid composition comprises the one or more fatty acid salts and, if present, the fatty acids in an amount of between about 25 to about 70 wt.-%, and in particular between about 28 wt.-% and about 65 wt.-%, relative to the total weight of the solid shaving aid composition.
5. The razor cartridge of any one of clauses 1 to 4, wherein the one or more polyols comprising from about 2 to about 8 carbon atoms comprises a saturated linear or branched C₂-C₈ polyol having between 1 and about 7, more specifically between about 2 and about 6, hydroxyl groups; or a mixture of several such compounds.
6. The razor cartridge of any one of clauses 1 to 5, wherein the one or more polyols comprising from about 2 to about 8 carbon atoms comprises a mixture of glycerol, sorbitol and at least one of propylene glycol, ethylene glycol, diethylene glycol, dipropylene glycol, and methylpropane diol glycol.
7. The razor cartridge of any one of clauses 1 to 6, wherein the solid shaving aid composition comprises one or more polyols comprising from about 2 to about 8 carbon atoms in an amount of between about 15 wt.-% to about 55 wt.-% and in particular between about 17 wt.-% and about 52 wt.-%, relative to the total weight of the solid shaving aid composition.
8. The razor cartridge of any one of clauses 1 to 7, wherein the non-ionic surfactant is selected from polyethoxylated, polypropoxylated and polyglycerolated fatty acids; alkylphenols; copolymers of ethylene oxide and of propylene oxide; condensates of ethylene oxide and of propylene oxide with fatty alcohols; polyethoxylated fatty amides; polyglycerolated fatty amides; polyethoxylated fatty amines; oxyethylenated fatty acid esters of sorbitan; fatty acid esters of sucrose; fatty acid esters of polyethylene glycol; and alkylpolyglycosides.
9. The razor cartridge of any one of clauses 1 to 8, wherein the solid shaving aid composition comprises, relative to the total weight of the solid shaving aid composition, between about 0.2 wt.-% to about 2.5 wt.-% and in particular between about 0.3 wt.-% to about 2.0 wt.-%, of the non-ionic surfactant.
10. The razor cartridge of any one of clauses 1 to 9, wherein the at least one zwitterionic surfactant comprises a positively charged nitrogen-based group, in particular a quaternary ammonium group, and a negatively charged group, in particular a sulfonate or carboxylate group.
11. The razor cartridge of any one of clauses of 1 to 10, wherein the at least one zwitterionic surfactant comprises a betaine and/or a sulfobetaine.
12. The razor cartridge of any one of clauses of 1 to 11, wherein the at least one zwitterionic surfactant comprises a betaine selected from (C₈-C₂₀)alkylbetaines, sulfobetaines, (C₈-C₂₀)alkylamido(C₁-C₆)alkylbetaines; and (C₈-C₁₀)alkylamido(C₁-C₆)alkylsulfobetaines which may optionally be oxyethylenated.
13. The razor cartridge of any one of clauses 1 to 12, wherein the at least one zwitterionic surfactant is pH-insensitive.
14. The razor cartridge of any one of clauses 1 to 13, wherein the at least one zwitterionic surfactant is present in amount of about 0.2 wt.-% to about 2.5 wt.-%, and in particular in amounts of about 0.3 wt.-% to about 2.0 wt.-%, relative to the total weight of the solid shaving composition.
15. The razor cartridge of any one of clauses 1 to 14, wherein the solid shaving aid composition comprises the water, in an amount between about 2.0 wt.-% to about 35 wt.-%, in particular about 5.0 wt.-% to about 25 wt.-%, relative to the total weight of the solid shaving aid composition.
16. The razor cartridge of any one of clauses 1 to 15, wherein the at least one butter is selected from the group consisting of cocoa butter, mango butter, shea butter and mixtures thereof.
17. The razor cartridge of any one of clauses 1 to 16, wherein the total content of butter(s) is between 0.5 wt.-% to 5.0 wt.-%, in particular about 0.5 % to about 4.0 wt.-%, relative to the total weight of the solid shaving aid composition.
18. The razor cartridge of any one of clauses 1 to 17, wherein the one or more oils comprises a humectant oil having a molecular weight of greater than about 500 g/mol; in particular a castor oil derivative.
19. The razor cartridge of clause 18, wherein the castor oil derivative comprises a castor oil esterified with 1, 2 or 3 dicarboxylic acid(s) having between about 3 and about 8 carbon atoms, and in particular comprises castoryl maleate.
20. The razor cartridge of clause 18 or clause 19, wherein the solid shaving aid composition comprises, relative to the total weight of the solid shaving aid composition, between about 0.1 wt.-% and about 4.0 wt.-%, more specifically about 0.4 wt.-% to about 3.0 wt.-%, in particular about 0.6 wt.-% to about 2.0 wt.-%, of the one or more oils.
21. The razor cartridge of any one of clauses 1 to 20, wherein the solid shaving aid composition comprises one or more silicone cross-polymers.
22. The razor cartridge of clause 21, wherein the one or more silicone cross-polymers comprises an emulsifying silicone cross-polymer.
23. The razor cartridge of clause 22, wherein one or more silicone cross-polymers comprises a first (di)methicone-based polymer chain which is crosslinked to a second (di)methicone-based polymer chain via at least one hydrophilic oligomer/polymer chain.
24. The razor cartridge of clause 23, wherein the first and/or second dimethicone-based polymer chain is selected from dimethicone and optionally substituted linear or branched saturated or unsaturated C₁-C₂₀ alkyl (di)methicone.
25. The razor cartridge of clause 23 or clause 24, wherein the hydrophilic oligomer/polymer chain is a C₁-C₄-alkyl polyether wherein the C₁-C₃-alkyl residues are optionally substituted with hydroxyl, hydroxyl-C₁-C₄-alkyl, C₁-C₄-alkyl ether groups or hydroxyl-C₁-C₄-alkylether groups, in particular polyethylene glycol, polypropylene glycol, polyglycerol, and polysorbate.
26. The razor cartridge of any one of clauses 1 to 25, wherein the one or more silicone crosspolymer comprises polyglycerine-modified cross-linked polymers, polyether-modified cross-linked polymers, polyether/alkyl co-modified cross-linked polymers, polyglycerine/alkyl co-modified cross-linked polymers, dimethicone/vinyl dimethicone cross-polymers, dimethicone/phenyl vinyl dimethicone cross-polymers, vinyl dimethicone/laury dimethicone cross-polymers, dimethicone crosspolymers, C₃₀₋₄₅-alkyl cetearyl dimethicone cross-polymers, cetearyl dimethicone cross-polymers, in particular dimethicone/vinyl dimethicone cross-polymers, vinyl dimethicone/lauryl dimethicone cross-polymers, vinyl dimethicone/lauryl dimethicone cross-polymers, lauryl dimethicone/polyglycerin cross-polymers, dimethicone/polyglycerin crosspolymers, polyethylene glycol/polypropoylene glycol-dimethicone cross-polymer; and mixtures thereof.
27. The razor cartridge of any one of clauses 1 to 26, wherein the one or more silicone crosspolymers is present in amounts of about 0.1 to about 1.0 wt.-%, more specifically between about 0.15 to about 0.7 wt.-%, and in particular between about 0.20 wt.-% and about 0.45 wt.-%, relative to the total weight of the solid shaving aid composition.
28. The razor cartridge of any one of clauses 1 to 27, wherein the solid shaving aid composition further comprises, relative to the total weight of the solid shaving aid composition, less than about 3.0 wt.-%, such as between about 0.1 wt.-% and about 2.0 wt.-%, or between about 0.2 wt.-% and about 1.5 wt.-%, of one or more sulfur-containing anionic surfactants, in particular anionic surfactants selected from alkyl sulfates, sulfosuccinates, alkyl benzene sulfanate, acyl methyl taurates, isethionates, monoglyceride sulfates and ether sulfanates.
29. The razor cartridge of any one of clauses 1 to 28, wherein the solid shaving aid composition further comprises between about 2.0 wt.-% and about 8.0 wt.-%, and in particular between 4.0 wt.-% and about 6.5 wt.-% of one or more bulking agent, relative to the total weight of the solid shaving aid composition.
30. The razor cartridge of clause 29, wherein the bulking agent comprises in particular sodium chloride.
31. The razor cartridge of any one of clauses 1 to 30, wherein the solid shaving aid composition comprises between about 0.04 wt.-% and about 0.8 wt.-%, and in particular between 0.06 wt.-% and about 0.6 wt.-% of one or more opacifying agents, relative to the total weight of the solid shaving aid composition.
32. The razor cartridge of clause 31, wherein the opacifying agent comprises an inorganic pigment, in particular titanium dioxide, and/or an styrene/acrylate copolymer.
33. The razor cartridge of any one of clauses 1 to 32, wherein the solid shaving aid composition comprises one or more chelating agents.
34. The razor cartridge of clause 33, wherein the chelating agent comprises a multidentate chelating agent, in particular pentasodium pentetate, tetrasodium etidronate, tetrasodium iminodissucinate, citric acid, sodium citrate or mixtures thereof.
35. The razor cartridge of clause 33 or 34, wherein the solid shaving aid composition comprises between about 0.01 wt.-% and about 2 wt.-%, and in particular between about 0.08 wt.-% and about 1.5 wt.-% of one or more multidentate chelating agents, relative to the total weight of the solid shaving aid composition.
36. The razor cartridge of any one of clauses 1 to 35, wherein the solid shaving aid composition further comprises, relative to the total weight of the solid shaving aid composition, between about 0.1 wt.-% and about 2.5 wt.-%, in particular between about 0.3 wt.-% and about 1.5 wt.-%, of a fragrance or mixture of fragrances.
37. The razor cartridge of any one of clauses 1 to 36, wherein the solid shaving aid composition further comprises, relative to the total weight of the solid shaving aid composition, between about 0.01 wt.-% and about 2.0 wt.-%, in particular between about 0.05 wt.-% and about 1.0 wt.-%, of an anti-oxidant or mixture of anti-oxidants.
38. The razor cartridge of any one of clauses 20k to claim 20 which defines the % oils. to 37, wherein the solid shaving aid composition further comprises, relative to the total weight of the solid shaving aid composition, between about 0.1 wt.-% to about 2 wt.-%, more specifically between about 0.2 wt.-% and about 1 wt.-%, and in particular between about 0.3 wt.-% and about 0.8 wt.-%, of squalane.
39. The razor cartridge of any one of clauses 1 to 38, wherein the solid shaving aid composition is substantially free or free of silicone oil and/or of silicone wax.
40. The razor cartridge of any one of clauses 1 to 39, wherein the solid shaving aid composition is substantially free or free of polar waxes, in particular substantially free or free of apolar waxes, such as polyethylene wax, microcrystalline wax, paraffin wax and ozokerite wax.
41. A solid shaving aid composition having a composition as defined in any one of clauses 1 to 40.
42. The solid shaving composition of clause 41, wherein the solid shaving composition is shaped into the form of a bar, more specifically a bar configured to be placed onto a razor cartridge, and in particular a bar having a length of between about 30 mm to about 80 mm, a width between about 2 mm and about 10 mm, and a depth of about 0.2 mm to about 4 mm.
43. Use of a solid shaving aid composition having a composition as defined in any one of clauses 1 to 42 in a multiplicity of facial or leg shaving operations, wherein the facial or leg shaving operations is repeated for at least about 8 times, specifically about 9 times, and in particular at least about 10 times, without replacing the solid shaving aid composition.

Although the embodiments of the present disclosure have been disclosed for illustrative purposes, those skilled in the art will appreciate that various modifications and alterations are possible, without departing from the spirit of the present disclosure. It is also to be understood that such modifications and alterations are incorporated in the scope of the present disclosure and the accompanying claims.

## Claims

1. A solid shaving aid (soap) composition comprising:
a housing having a front edge and a rear edge;
one or more shaving blades disposed between the front edge and the rear edge; and
at least one shaving bar disposed in front of the blade(s) and/or rear of the blade(s) which comprises a solid shaving aid composition;
wherein the solid shaving aid composition comprises, relative to the total weight of the solid shaving aid composition:
about 25 wt.-% to about 75 wt.-% of a soap base consisting of one or more fatty acid salts having from about 8 to about 24 carbon atoms and optionally one or more fatty acids having from about 8 to about 24 carbon atoms,
about 15 wt.-% to about 60 wt.-% of one or more polyols comprising from about 2 to about 8 carbon atoms,
about 0.1 wt.-% to about 3.0 wt.-% of one or more non-ionic surfactants,
about 0.1 wt.-% to about 3.0 wt.-% of one or more zwitterionic surfactants,
one or more butters, and
one or more oils.

2. The razor cartridge of claim 1, wherein the non-ionic surfactant is selected from polyethoxylated, polypropoxylated and polyglycerolated fatty acids; alkylphenols; copolymers of ethylene oxide and of propylene oxide; condensates of ethylene oxide and of propylene oxide with fatty alcohols; polyethoxylated fatty amides; polyglycerolated fatty amides; polyethoxylated fatty amines; oxyethylenated fatty acid esters of sorbitan; fatty acid esters of sucrose; fatty acid esters of polyethylene glycol; and alkylpolyglycosides.

3. The razor cartridge of any one of claims 1 to 2, wherein the solid shaving aid composition comprises, relative to the total weight of the solid shaving aid composition, between about 0.2 wt.-% to about 2.5 wt.-% and in particular between about 0.3 wt.-% to about 2.0 wt.-%, of the non-ionic surfactant.

4. The razor cartridge of any one of claims 1 to 3, wherein the at least one zwitterionic surfactant comprises a positively charged nitrogen-based group, in particular a quaternary ammonium group, and a negatively charged group, in particular a sulfonate or carboxylate group.

5. The razor cartridge of any one of claims of 1 to 4, wherein the at least one zwitterionic surfactant comprises a betaine and/or a sulfobetaine.

6. The razor cartridge of any one of claims of 1 to 5, wherein the at least one zwitterionic surfactant comprises a betaine selected from (C₈-C₂₀)alkylbetaines, sulfobetaines, (C₈-C₂₀)alkylamido(C₁-C₆)alkylbetaines; and (C₈-C₁₀)alkylamido(C₁-C₆)alkylsulfobetaines which may optionally be oxyethylenated.

7. The razor cartridge of any one of claims 1 to 6, wherein the at least one zwitterionic surfactant is pH-insensitive.

8. The razor cartridge of any one of claims 1 to 7, wherein the at least one zwitterionic surfactant is present in amount of about 0.2 wt.-% to about 2.5 wt.-%, and in particular in amounts of about 0.3 wt.-% to about 2.0 wt.-%, relative to the total weight of the solid shaving composition.

9. The razor cartridge of any one of claims 1 to 8, wherein the at least one butter is selected from the group consisting of cocoa butter, mango butter, shea butter and mixtures thereof.

10. The razor cartridge of any one of claims 1 to 9, wherein the total content of butter(s) is between 0.5 wt.-% to 5.0 wt.-%, in particular about 0.5 % to about 4.0 wt.-%, relative to the total weight of the solid shaving aid composition.

11. The razor cartridge of any one of claims 1 to 10, wherein the one or more oils comprises a humectant oil having a molecular weight of greater than about 500 g/mol; in particular a castor oil derivative.

12. The razor cartridge of claim 11, wherein the castor oil derivative comprises a castor oil esterified with 1, 2 or 3 dicarboxylic acid(s) having between about 3 and about 8 carbon atoms, and in particular comprises castoryl maleate.

13. The razor cartridge of claim 11 or claim 12, wherein the solid shaving aid composition comprises, relative to the total weight of the solid shaving aid composition, between about 0.1 wt.-% and about 4.0 wt.-%, more specifically about 0.4 wt.-% to about 3.0 wt.-%, in particular about 0.6 wt.-% to about 2.0 wt.-%, of the one or more oils.

14. A solid shaving aid composition having a composition as defined in any one of claims 1 to 13.

15. The solid shaving composition of claim 14, wherein the solid shaving composition is shaped into the form of a bar, more specifically a bar configured to be placed onto a razor cartridge, and in particular a bar having a length of between about 30 mm to about 80 mm, a width between about 2 mm and about 10 mm, and a depth of about 0.2 mm to about 4 mm.
